# EUROPÄISCHE PATENTANMELDUNG

(11) **EP 3 034 605 A1**
(43) Veröffentlichungstag der Anmeldung: **22.06.2016**
(21) Anmeldenummer: 15000238.4
(22) Anmeldetag: 27.01.2015
(51) Int. Cl.: C12N 1/12, A01G 33/00, C12M 1/00, C12M 1/34, B01D 53/62, B01D 53/84

(54) **CO2-Dosierung für die Algenproduktion**

(30) Priorität: 17.12.2014 DE 102014018901
(71) Anmelder: Linde Aktiengesellschaft, 80331 München (DE)
(72) Erfinder: van de Ven, Joost, 5235 DC's-Hertogenbosch (NL); Dullstein, Stefan, 85375 Neufahrn (DE)
(74) Vertreter: Gellner, Bernd

(57) **Zusammenfassung**

Die vorliegende Erfindung betrifft ein Verfahren zur Vermehrung eines autotroph CO₂-assimilierenden Organismus, umfassend die Schritte Bereitstellen eines Reaktionsraumes umfassend ein wässriges Kulturmedium, Einleiten eines CO₂-haltigen Gasstromes in das Kulturmedium, Kultivieren des autotroph CO₂-assimilierenden Organismus im Kulturmedium. Erfindurigsgemäß ist dabei vorgesehen, dass das Kulturmedium einen pH-Wert von 8,5 bis 11 aufweist, und der CO₂-haltige Gasstrom kontinuierlich in das Kulturmedium eingeleitet wird, wobei insbesondere vom CO₂-Assimilierenden Organismus nicht umgesetztes CO₂ als Hydrogencarbonat oder Carbonat im Kulturrüedium gelöst wird.

## Beschreibung

Die Erfindung betrifft ein Verfahren zur Vermehrung bzw. Kultivierung autotroph CO₂-assmilierender Organismen.

Ein solches Verfahren umfasst die Schritte: Bereitstellen eines Reaktionsraumes umfassend ein wässriges Kulturmedium, Einleiten eines CO₂-haltigen Gasstromes in das Kulturmedium und Kultivieren des atrophic CO₂-assimilierenden Organismus im Kulturmedium.

Biomasse kann durch verschiedenste Verfahren und Mittel hergestellt werden. Eine Möglichkeit zur Herstellung von Biomasse, insbesondere Algen, ist der Einsatz von Algenfarmen, wobei Algen insbesondere in offenen Becken kultiviert werden. Zur Aufrechterhaltung der Lebensprozesse und insbesondere für ihr Wachstum benötigen Algen neben Sonnenlicht vor allem CO₂, das in der Photosynthese letztendlich zu Kohlenhydraten umgesetzt wird.

In aus dem Stand der Technik bekannte Algenzuchtanlagen und -verfahren wird CO₂ periodisch ausschließlich tagsüber in die Zuchtbecken eingespeist. Die Einspeisung erfolgt in der Regel alle 30 Minuten, um eine die für die Algenproduktion notwendige pH-Stabilität zu erreichen.

Ein Nachteil der bekannten Verfahren ist es, dass CO₂ nur tagsüber, also im Durchschnitt nur 12h pro Tag, eingespeist wird, was das Wachstum und damit die Biomasseausbeute in dieser Hinsicht limitiert.

Ein weiterer Nachteil der bekannten Verfahren ist es, dass die periodische Einspeisung von CO₂ eine Vielzahl von CO₂-Einspeisungsstellen erfordert, damit während eines Einspeisungszyklus genügen CO₂ in das Zuchtbecken eingebracht und gleichmäßig verteilt werden kann.

Basierend darauf ist die Aufgabe der Erfindung ein einfaches und wirtschaftlich tragbares Verfahren zur Algenzucht zur Verfügung zu stellen.

Diese Aufgabe wird durch ein Verfahren mit den Merkmalen des Anspruchs 1 gelöst. Vorteilhafte Ausführungsformen sind in den Unteransprüchen angeben und werden unten beschrieben.

Danach ist vorgesehen, dass das Kulturmedium einen pH-Wert von 8,5 bis 11 aufweist, und der CO₂-haltige Gasstrom kontinuierlich in das Kulturmedium eingeleitet wird, wobei insbesondere vom CO₂-assimilierenden Organismus (insbesondere Algen) nicht umgesetztes CO₂ als Hydrogencarbonat oder Carbonat im Kulturmedium gelöst wird bzw. in gelöster Form vorliegt.

Das vorliegende Verfahren ist nicht auf die Anwendung in Open-Pond-Anlagen beschränkt und kann z.B. auch in Photobioreaktoren angewendet werden.

Die Sauerstoffversorgung der Algen kann ganz oder teilweise durch die Eigenproduktion der Algen gewährleistet werden. Hierbei können die Algen CO₂ (HCO₃-) aus dem Wasser entnehmen und O₂ produzieren.

Das Kulturmedium bzw. Zuchtmedium kann Nahrungsmittel wie Stickstoffverbindungen und/oder Phosphorverbindungen enthalten sowie ggf. andere Spurenelemente.

Vorteilhafterweise ist bei dem erfindungsgemäßen Verfahren eine geringe CO₂-Zufuhrleistung nötig, da die Tagesmenge CO₂ über 24 Stunden verteilt zugeführt werden kann, anstatt nur während des Tages bzw. während des Tageslichtes. Daher können vorteilhafterweise zur Einspeisung notwendige Rohrleitungen oder Pumpen kleiner dimensioniert werden. Insbesondere sind dadurch weniger CO₂-Einspeisungsstellen notwendig. Auch wird durch die nun mögliche verringerte CO₂-Zufuhrleistung der Aufwand für die Vorbehandlung des CO₂ bzw. des CO₂-haltigen Gasstroms verringert. Weiterhin wird vorteilhafterweise das gepufferte CO₂ mit dem Wasser im Becken verteilt, woraus auch unter anderem ein geringerer Installationsaufwand resultiert. Ferner ist das Verfahren weniger anfällig für Schwankungen in der CO₂-Versorgung, da das CO₂ zwar kontinuierlich, aber in geringeren Mengen eingespeist wird und nicht, wie im Stand der Technik bekannt, in großen Mengen in einer relativ kurzen Zeitspanne. Daher muss im erfindungsgemäßen Verfahren CO₂ nicht aufwändig vorrätig gehalten werden, um auftretende Schwankungen kompensieren zu können. Insgesamt werden der Installationsaufwand und die Betriebskosten für Zuchtanlagen durch das erfindungsgemäße Verfahren verringert.

Als autotroph CO₂-assimilierender Organismus wird im Sinne der Erfindung insbesondere ein Organismus bezeichnet, der in einem wässrigen Medium kultivierbar ist und CO₂ als Kohlenstoffquelle zum Aufbau organischer Moleküle aufnimmt, insbesondere im Rahmen photosynthetischer Prozesse (z.B. Algen).

Als Vermehrung oder Kultivierung bezeichnet man im Sinne der vorliegenden Erfindung insbesondere Vorgänge, bei denen sich die Gesamtmasse der individuellen Strukturen eines Organismus wie etwa Zellen, Zellorganellen, Zellgewebe, Gewebe oder Organe vermehrt, d. h. insbesondere, dass der Organismus wächst, oder Vorgänge, bei denen sich der Organismus geschlechtlich oder ungeschlechtlich vermehrt. Insbesondere führt eine Vermehrung bzw. Kultivierung im Sinne der Erfindung zu einer Zunahme der Biomasse der CO₂-assimilierenden Organismen.

Die Vermehrung bzw. Kultivierung umfasst insbesondere zumindest eine CO₂-fixierende Reaktion im autotroph CO₂-assimilierenden Organismus, bei der das eingespeiste anorganische CO₂ in ein anderes Molekül, insbesondere ein organisches Molekül, umgesetzt wird. Beispiele für solche CO₂-fixierenden Reaktionen umfassen, ohne darauf beschränkt zu sein,
- die Umsetzung von CO₂ mit Ribulose-1,5-bisphosphat zu 3-Phosphoglycerat (eine Reaktion des Calvinzyklus),
- die Umsetzung von CO₂ als Bicarbonat mit Phosphoenolpyruvat zu Oxalacetat (eine CO₂-fixierende Reaktion der sogenannten C4-Pflanzen),
- die Umsetzung von CO₂ mit Acetyl-CoA zu Pyruvat (eine Reaktion des reduktiven Citratzyklus),
- die Umsetzung von CO₂ zu Acetyl-CoA (eine Reaktion des reduktiven Acetyl-CoA-Weges),
- die Umsetzung von CO₂ in Form von Bicarbonat über Propionyl-CoA und Glyoxylat zu Pyruvat (eine Reaktion des 3-Hydroxypropionatzyklus)
- die Umsetzung von CO₂ mit Acetyl-CoA über 3-Hydroxypropionat und 4-Hydroxybutyrat zu Acetyl-CoA (eine Reaktion des 3-Hydroxypropionat/4-Hydroxybutyratzyklus).

In einer Ausführungsform der Erfindung ist der autotroph CO₂-assimilierende Organismus ein photoautotropher Organismus, d.h., ein Organismus, der Licht als Energiequelle zum Aufbau der organischen Moleküle verwenden kann.

In einer Ausführungsform der Erfindung ist der autotroph CO₂-assimilierende Organismus eine Alge oder ein Cyanobakterium. In einer Ausführungsform der Erfindung ist der autotroph CO₂-assimilierende Organismus eine Grünalge (*Chlorophyta*), eine Rotalge (*Rhodphyta*) oder eine Braunalge (*Pheaophyta*). Insbesondere können Algen verwendet werden, die bei den vorgesehenen höheren pH-Werten wachsen können. Der vergleichsweise höhere pH-Wert hat auch den Vorteil, dass einige Organismen, die Algen konsumieren, bei den vergleichsweise hohen pH-Werten nicht existieren können. Parallel ist auch eine zusätzliche Kultivierung von Wasserpflanzen denkbar.

In einer Ausführungsform der Erfindung enthält der CO₂-haltige Gasstrom CO₂ in einer Konzentration von 4 % (v/v) bis 100 % (v/v).

In einer Ausführungsform ist der CO₂-haltige Gasstrom ein Abgasstrom eines Verbrennungsprozesses, in welchem kohlenstoffhalte Verbindung oxidiert werden, insbesondere mit Luft oder elementaren Sauerstoff.

In einer Ausführungsform wird der CO₂-haltige Gasstrom durch ein CO₂-Reservoir bereitgestellt. Das CO₂-Reservoir kann eine CO₂-Versorgungsleitung oder ein CO₂-Speicher sein, wobei CO₂ in fester, flüssiger oder gasförmiger Form im CO₂-Speicher vorliegen kann. In einer Ausführungsform der Erfindung wird flüssiges CO₂ dem CO₂-Speicher entnommen, in einem Verdampfer in die Gasform überführt und das resultierende gasförmige CO₂ bzw. der resultierende CO₂-haltige Gasstrom in das Kulturmedium eingeleitet.

In einer Ausführungsform der Erfindung ist der Reaktionsraum ein offenes Becken, wobei das offene Becken insbesondere dazu ausgebildet ist, eine Beleuchtung des Kulturmediums mit natürlichem Licht oder künstlichen Licht zu ermöglichen, und insbesondere einen Stoffaustausch des Kulturmediums mit der Umwelt zu ermöglichen. Der Begriff "Stoffaustausch" bezieht sich insbesondere auf die Abgabe von Sauerstoff aus dem Kulturmedium an die Umwelt.

In einer Ausführungsform der Erfindung umfasst das offene Becken höchstens eine Injektionsstelle für das Einleiten des CO₂-haltigen Gasstroms je 7ha der Pegeloberfläche des Kulturmediums. Die Pegeloberfläche bezeichnet insbesondere bei offenen Becken die Oberfläche des Kulturmediums zur umgebenden Atmosphäre hin (z.B. Wasseroberfläche).

Eine Injektionsstelle im Sinne der Erfindung bezeichnet insbesondere einen Bereich des offenen Beckens bzw. Reaktionsraumes, der durch eine einzelne Versorgungsleitung gespeist wird, und über die der CO₂-haltige Gasstrom in das Kulturmedium eingeleitet wird. Vorzugsweise findet die Einleitung des CO₂-haltigen Gasstroms am Boden des Beckens statt.

In einer Ausführungsform der Erfindung ist die Injektionsstelle als ein perforierter Begasungsschlauch ausgebildet. In einer weiteren Ausführungsform der Erfindung ist die Injektionsstelle als Begasungsmatte ausgebildet, wobei ein oder mehrere Begasungsschläuche am Beckenboden des offenen Beckens installiert sind. Ein Vorteil der Verwendung von perforierten Begasungsschläuchen ist es, dass damit der CO₂-haltige Gasstrom gleichmäßig und feinblasig in das Kulturmedium eingeleitet werden kann. Vorteilhafterweise besteht der perforierte Begasungsschlauch aus einem chemikalienresistenten Elastomer.

In einer Ausführungsform der Erfindung ist die Injektionsstelle als eine Kugelkopfdüse ausgebildet.

Weiterhin kann CO₂ auch über einen sogenannten Plattenbelüfter (z.B. Dome Injector), wie er z.B. auch in Kläranlagen eingesetzt wird, in das Zuchtmedium eingespeist werden. Der Vorteil dieser Form des CO₂-Eintrags ist der niedrige Druckbedarf bei der Eindüsung von CO₂ im Vergleich zu perforierten Schläuchen.

Bei Systemen, in denen das Wasser im Kreislauf gepumpt wird, ist grundsätzlich auch die Einbringung von CO₂ in das Zuchtmedium über einen Reaktor denkbar.
Im Vergleich zur Kugelkopfdüse kann eine höhere Eintragseffizienz erwartet werden, da sich so mehr CO₂ pro Wasservolumen zudosieren lässt. Allerdings wird eine Pumpe benötigt, die das Zuchtmedium aus dem Becken in den Reaktor und wieder zurück in das Becken pumpt.

Weitere Einzelheiten und Vorteile der Erfindung sollen durch die nachfolgende Figurenbeschreibung von Ausführungsbeispielen anhand der Figuren erläutert werden.

Es zeigen:
- Fig. 1: eine schematische Darstellung der CO₂-Bereitstellung und -Assimilierung im erfindungsgemäßen Verfahren; und
- Fig. 2: eine bevorzugte Ausführungsform der Erfindung.

### Beispiele:

Bei herkömmlichen, aus dem Stand der Technik bekannten Algenzuchtverfahren, wird die CO₂-Versorgung der Algen an das Tageslicht angepasst. Die Algen wachsen nur während des Tages und verbrauchen daher CO₂ im Durchschnitt nur 12h pro Tag. In den meisten bekannten Algenzuchtsystemen wird daher CO₂ oder Rauchgas nur über 12h pro Tag in die Algenzucht eingebracht. Für große Algenzuchtbecken (>5 ha) sind eine Vielzahl von Einspeisungsstellen für CO₂ oder Rauchgas vorgesehen, um das CO₂ homogen im Zuchtbecken zu verteilen. Dies erfordert einen erheblichen apparativen Aufwand, da unter anderem die Einspeisungsstellen in Regel unabhängig voneinander durch viele Versorgungsleitungen gespeist werden müssen.

Zur Senkung der Installations- und Betriebskosten wäre daher eine kontinuierliche CO₂-Einspeisung, also 24h am Tag, vorteilhaft, da dadurch die Zahl der Einspeisungsstellen und der dazugehörigen Versorgungsleitungen stark reduzierte werden könnte. Nachteilig ist jedoch, dass nicht umgesetztes CO₂ potentiell aus dem Zuchtmedium ausgasen kann.

Erfindungsgemäß ist daher vorgesehen, CO₂ 13 kontinuierlich über 24h pro Tag in das Kulturmedium 21 im Algenzuchtbecken bzw. im Reaktionsraum B einzuspeisen, das bei einem pH-Wert von 8.5 bis 11 betrieben wird. Das eingespeiste CO₂ 13 wird dabei zur Pufferung des Kulturmediums 21 verwendet. Nicht umgesetztes CO₂ wird während der Dunkelheit durch das erfindungsgemäße CO₂-Puffersystem über Carbonate und Hydrogencarbonate in gelöster Form gehalten. Die Verwendung des Puffersystems erlaubt eine kontinuierliche CO₂-Einspeisung 13 in das Zuchtbecken, und somit eine Reduzierung der Anzahl und der Größe der Einspeisungsstellen 11, der Größe der Einspeisungsausrüstung, der CO₂⁻ bzw. Rauchgasvorbehandlung und des Leitungssystems. Durch die Verwendung des Puffersystems gast CO₂ nicht aus, sondern verbleibt in gelöster Form im Zuchtmedium. CO₂ 13 kann in einer Konzentration von 4% (v/v) bis 100 % (v/v) in Form von beispielsweise Rauchgas oder in reiner Form in das Kulturmedium (auch als Zuchtmedium bezeichnet) 21 eingespeist werden.

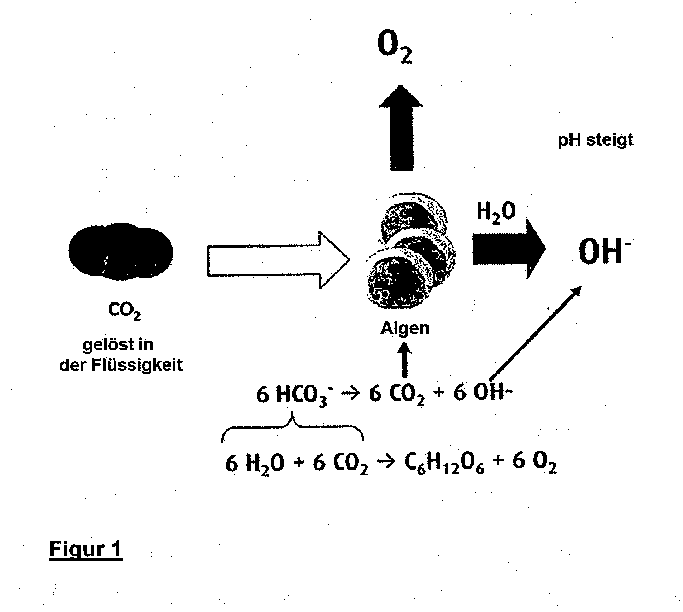

Figur 1 zeigt schematische die Vorgänge der CO₂-Assimilation. CO₂ liegt in gelöster Form als Hydrogencarbonat im Zuchtmedium. Die Algen nehmen das CO₂ aus dem Hydrogencarbonat oder dem Carbonat auf und setzen es während der Photosynthese, insbesondere in der Dunkelreaktion, zu Glukose und Sauerstoff um. Bei der Freisetzung von CO₂ aus Hydrogencarbonat entstehen Hydroxidionen, die den pH-Wert des Zuchtmediums erhöhen.

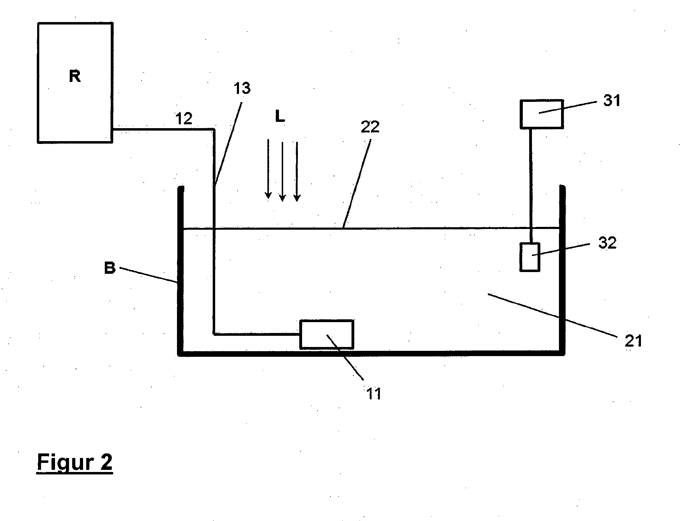

Figur 2 zeigt eine bevorzugte Anlagen- und Verfahrensform der Erfindung. Ein Zuchtbecken B ist als ein nach oben offenes Becken ausgebildet und umfasst ein Zucht- bzw. Kulturmedium 21, in welchen Algen vermehrt werden sollen. Das Zuchtmedium 21 kann dabei von oben mit natürlichem oder künstlichem Licht L beleuchtet werden. Die Menge an Licht L wie etwa Sonnenlicht, die die Algen im Zuchtmedium 21 aufnehmen können, ist dabei unter anderem von der beleuchtbaren Fläche, der Pegeloberfläche 22 des Zuchtmediums 21, abhängig. Da für das Wachstum genötigte CO₂ 13 wird aus einem CO₂-Reservoir R über eine Versorgungsleitung 12 in eine CO₂-Einspeisungsstelle 11 geführt und über diese 11 dann in das Zuchtmedium 21 geleitet. Das Zuchtmedium 21 weist erfindungsgemäß einen pH-Wert von 8,5 bis 11 auf, so dass nicht umgesetztes CO₂ nicht ausgast, sondern in löslicher Form vorliegt. Der pH-Wert des Zuchtmediums 21 kann dabei über eine pH-Elektrode 32, die in das Zuchtmedium 21 hineinragt, und an ein daran angeschlossenes pH-Meter 31 kontrolliert werden. Vorteilhafterweise ist die CO2-Einspeisungsstelle als perforierter Begasungsschlauch oder als Kugelinjektor ausgebildet. Es ist auch möglich, CO₂ über andere Düsen, wie z.B. Venturi-Düsen in das Medium 21 einzuspeisen.

**Bezugszeichenliste**

| | |
|---|---|
| R | CO₂-Reservoir |
| B | Zuchtbecken |
| L | Lichtquelle bzw. Licht |
| 11 | CO₂-Einspeisungsstelle |
| 12 | Versorgungsleitung für CO₂-Einspeisungsstelle |
| 13 | CO₂-haltiger Gasstrom |
| 21 | Zucht/Kulturmedium |
| 22 | Pegeloberfläche des Zucht/Kulturmediums |
| 31 | pH-Meter |
| 32 | pH-Elektrode |

## Patentansprüche

1. Verfahren zur Vermehrung eines autotroph CO₂-assimilierenden Organismus, umfassend die Schritte:
- Bereitstellen eines Reaktionsraumes (B) umfassend ein wässriges Kulturmedium (21),
- Einleiten eines CO₂-haltigen Gasstromes (13) in das Kulturmedium (21),
- Kultivieren des autotroph CO₂-assimilierenden Organismus im Kulturmedium (21),
**dadurch gekennzeichnet, dass**
das Kulturmedium (21) einen pH-Wert von 8,5 bis 11 aufweist, und der CO₂-haltige Gasstrom (13) kontinuierlich in das Kulturmedium (21) eingeleitet wird, wobei insbesondere vom CO₂-assimilierenden Organismus nicht umgesetztes CO₂ als Hydrogencarbonat oder Carbonat im Kulturmedium gelöst wird.

2. Verfahren nach Anspruch 1, **dadurch gekennzeichnet, dass** der CO₂-assimilierende Organismus eine Alge oder ein Cyanobakterium ist.

3. Verfahren nach Anspruch 1 oder 2, **dadurch gekennzeichnet, dass** der CO₂-haltige Gasstrom CO₂ (13) in einer Konzentration von 4 % (v/v) bis 100 % (v/v) enthält.

4. Verfahren nach einem der Ansprüche 1 bis 3, **dadurch gekennzeichnet, dass** der CO₂-haltige Gasstrom (13) durch ein CO₂-Reservoir (R) oder einen Verbrennungsprozess bereitgestellt wird, bei welchem insbesondere zumindest eine kohlenstoffhaltige Verbindung oxidiert wird, insbesondere durch Luft oder elementaren Sauerstoff.

5. Verfahren nach einem der Ansprüche 1 bis 4, **dadurch gekennzeichnet, dass** der Reaktionsraum (B) ein offenes Becken ist.

6. Verfahren nach Anspruch 5, **dadurch gekennzeichnet, dass** das offene Becken (B) höchstens eine Injektionsstelle (11) für das Einleiten des CO₂-haltigen Gasstroms (13) je 7ha der Pegeloberfläche (22) des Kulturmediums (21) umfasst.

7. Verfahren nach einem der Ansprüche 1 bis 6, **dadurch gekennzeichnet, dass** die Injektionsstelle (11) als ein perforierter Begasungsschlauch oder als eine
Kugelkopfdüse ausgebildet ist.
